Europäisches Patentamt

European Patent Office

Office européen des brevets

(11) Veröffentlichungsnummer: **0 391 080**
**A2**

## (12) EUROPÄISCHE PATENTANMELDUNG

(21) Anmeldenummer: 90104291.1

(51) Int. Cl.⁵: **C12N 5/00, C12N 15/58**

(22) Anmeldetag: 06.03.90

Der (Die) Mikroorganismus (Mikroorganismen) ist (sind) bei der Deutschen Sammlung von Mikroorganismen und Zellkulturen unter der (den) Nummer(n) DSM 4720 und DSM 4721 und bei PHLS Centre for Applied Microbiology & Research unter der Nummer 88072103 hinterlegt worden.

(30) Priorität: 07.03.89 DE 3907304
09.08.89 DE 3926339
14.08.89 DE 3926948

(43) Veröffentlichungstag der Anmeldung:
10.10.90 Patentblatt 90/41

(84) Benannte Vertragsstaaten:
AT BE CH DE DK ES FR GB GR IT LI LU NL SE

(71) Anmelder: **BOEHRINGER MANNHEIM GMBH**
**Sandhofer Strasse 112-132**
**D-6800 Mannheim-Waldhof(DE)**

(72) Erfinder: **Stockinger, Hubertus, Dr. rer. nat.**
**Unterholzstrasse 22**
**D-8122 Penzberg(DE)**
Erfinder: **Kionka, Christine, Dr. rer. nat.**
**Einbach 120 1/2**
**D-8170 Bad Tölz(DE)**
Erfinder: **Weidle, Ulrich, Dr. rer. nat.**
**Landwehrstrasse 56**
**D-8000 München 2(DE)**

(74) Vertreter: **Huber, Bernhard, Dipl.-Chem. et al**
**Möhlstrasse 22 Postfach 860 820**
**D-8000 München 86(DE)**

(54) Verfahren zur Herstellung von zweikettigem t-PA.

(57) Zur Herstellung von zweikettigem t-PA durch Kultivierung von eukaryontischen Zellen, die mit einem das t-PA-Gen enthaltenden Vektor transfiziert sind, inkubiert man nach Erreichen der stationären Wachstumsphase der Zellen die Kultur oder den Kulturüberstand mit einem Nachinkubationsmedium, welches Serum oder Plasma von Pferd, Rind oder foetalem Kalb und ein Fibrinogenderivat enthält, und gewinnt den t-PA aus dem Medium.

EP 0 391 080 A2

## Verfahren zur Herstellung von zweikettigem t-PA

Die Erfindung betrifft ein Verfahren zur Herstellung von zweikettigem t-PA durch Kultivierung von eukaryontischen Zellen, die mit einem das t-PA-Gen enthaltenden Vektor transfiziert sind.

Der Gewebetyp-Plasminogenaktivator t-PA ist eine Serinprotease mit einem Molekulargewicht von 68000 Dalton. Ihre Funktion liegt in der Umwandlung von Plasminogen zu Plasmin, das ebenfalls eine Serinprotease mit einem breiten Wirkungsspektrum ist. Wie andere Serinproteasen auch wird t-PA als eine einzige Polypeptidkette synthetisiert und dann durch Spaltung an Position 257/258 in eine zweikettige Form umgewandelt. Die beiden Ketten der zweikettigen Form werden durch eine Disulfidbrücke zusammengehalten. Die leichtere Kette enthält den enzymatisch aktiven Teil, während die schwerere Kette die sogenannten Finger-, EGF- und Kringeldomänen enthält. Durch Untersuchungen, bei denen das Arginin an der Aminosäureposition 257 eliminiert wurde, wurde festgestellt, daß die Spaltung an dieser Stelle für die enzymatische Aktivität essentiell ist (Mol. Biol. Meth. 3 (1986) 449-457). Bei den bisher bekannten Herstellungsformen wird eine Mischung aus ein- und zweikettigem t-PA hergestellt. Für eine therapeutische Anwendung ist es jedoch vorteilhaft, von Abbauprodukten freies reines entweder ein- oder zweikettiges t-PA ohne großen Aufwand herstellen zu können.

Verfahren zur Herstellung von einkettigem t-PA sind beispielsweise aus EP 151 996 A2 und Biochem. J. 226 (1985) 631-636 bekannt, wobei rekombinanter einkettiger t-PA in eukaryontischen Zellen in Gegenwart von Aprotinin hergestellt wird. Hierbei wird nahezu vollständig einkettiger t-PA erhalten. Ebenfalls aus der EP 151 996 A2 ist es bekannt, t-PA in nahezu vollständig zweikettiger Form herzustellen. Hierzu wird der Kulturüberstand von Eukaryontenzellen, in denen rekombinanter t-PA hergestellt wird, einer Enzymbehandlung, nämlich einer Behandlung mit Plasmin, Kallikrein, Trypsin, aktiviertem Faktor XI oder XII unterworfen. Die Beimischungen an einkettigem t-PA sind so gering, daß die Reinheit für viele Anwendungsarten ausreicht.

Für eine therapeutische Anwendung ist jedoch unbedingt zu fordern, daß t-PA entweder in reiner einkettiger Form oder in reiner zweikettiger Form hergestellt werden kann. Die Herstellung von t-PA in reiner einkettiger Form ist in der DE 38 29 244 beschrieben. Der vorliegenden Erfindung lag die Aufgabe zugrunde, es zu ermöglichen, zweikettigen t-PA in reiner Form herzustellen.

Erfindungsgemäß wird diese Aufgabe gelöst durch ein Verfahren zur Herstellung von zweikettigem t-PA durch Kultivierung von eukaryontischen Zellen, die mit einem das t-PA-Gen enthaltenden Vektor transfiziert sind, welches dadurch gekennzeichnet ist, daß man nach Erreichen der stationären Wachstumsphase der Zellen die Kultur oder den Kulturüberstand mit einem Nachinkubationsmedium, welches Serum oder Plasma von Pferd, Rind oder foetalem Kalb und ein Fibrinogenderivat enthält, inkubiert und den t-PA aus dem Medium gewinnt.

Die Erfindung beruht auf der überraschenden Feststellung, daß durch Zugabe von Serum oder Plasma von Rind, Pferd oder foetalem Kalb und einem Fibrinogenderivat zur Kultur, nachdem die stationäre Wachstumsphase erreicht ist, oder zum Kulturüberstand, der eine Mischung aus ein- und zweikettigem t-PA enthält, nach Inkubation zweikettigen t-PA in reiner Form hergestellt werden kann. Es ist hierbei im Rahmen der Erfindung auch möglich, reinen einkettigen t-PA, der z.B. gemäß der DE 38 29 244 hergestellt wurde, durch Behandlung mit Serum oder Plasma von Rind, Pferd oder foetalem Kalb und mit einem Fibrinogenderivat in die reine zweikettige Form zu überführen. Im Rahmen der Erfindung umfaßt der Begriff "Kulturüberstand" daher auch reinen einkettigen t-PA. Weiter sind im Rahmen der Erfindung unter dem Begriff t-PA auch Muteine bzw. Derivate des Proteins zu verstehen. Zur Herstellung solcher Muteine bzw. Derivate wird dann im erfindungsgemäßen Verfahren ein entsprechendes, dafür kodierendes Gen transfiziert.

Unter Fibrinogenderivaten sind im Rahmen der Erfindung Spaltprodukte des Fibrinogens zu verstehen, die beispielsweise in Eur. J. Biochem. 172 (1988), 399-404, J. Biochem. 262 (1987) 5944-5946 und Eur. J. Biochem. 166 (1987) 393-397 oder BBA 748 (1983) 86-92 beschrieben sind. In einer besonders bevorzugten Ausführungsform der Erfindung wird ein Fibrinogenfragment verwendet, das in den genannten Literaturstellen als FCB2 bezeichnet wird.

Als Medien zur Kultivierung der eukaryontischen Zellen können im Rahmen der Erfindung alle serumhaltigen oder serumfreien Medien verwendet werden, die zur Züchtung von Eukaryonten geeignet sind, z.B. RPMI 1640 (H.J. Morton, In Vitro 6 (1970) 89), DMEM (Dulbecco - modifi ziertes minimalessentielles Medium, J.W. Gooding, J. Immunol. Methods 39 (1980) 285) oder F12 (R. G. Ham, Proc. Natl. Acad. Sci. USA 53 (1965), 288) oder ein Medium nach DE 38 01 236 (mit oder ohne Serum).

Besonders bevorzugt wird im Rahmen der Erfindung eine Mischung von DMEM- und F12-Medium etwa im Verhältnis 1:1 eingesetzt. Ganz besonders bevorzugt wird das in der deutschen Patentanmeldung DE 38 01 236 der gleichen Anmelderin beschriebene Medium verwendet.

Zur Aufrechterhaltung der Stabilität der Plasmidtransformation der Zellen wird vorzugsweise ein Vektor verwendet, der zusätzlich zu dem t-PA-Gen ein selektionierbares Gen enthält, und dem Medium ein dem Vektor entsprechendes Selektionsmittel hinzugefügt. Geeignete Selektionsmittel sind beispielsweise Neomycin, Hygromycin, Mycophenolsäure, Hypoxanthin, Xanthin, Aminopterin oder aber auch Methotrexat und Derivate davon. Das erfindungsgemäß verwendete Medium enthält vorzugsweise kein Aprotinin, da festgestellt wurde, daß Aprotinin im Gegensatz zu den Lehren des Standes der Technik sich nachteilig auf die Stimulierbarkeit des gebildeten t-PA auswirkt. Mediumwechsel und Kulturbedingungen sind dem Fachmann bekannt und können in an sich üblicher Weise durchgeführt werden.

Nach Erreichen der stationären Wachstumsphase, d.h. wenn die maximale Zelldichte erreicht ist, wird die Kultur oder der Kulturüberstand mit einem Nachinkubationsmedium inkubiert, welches Serum oder Plasma von Rind, Pferd oder foetalem Kalb sowie ein Fibrinogenderivat enthält. Vorzugsweise wird foetales Kälberserum verwendet. Das Serum oder Plasma wird vorzugsweise in einer Menge von 0,01 bis 20 % der Kultur oder dem Kulturüberstand zugesetzt.

Im Rahmen der Erfindung wird das Fibrinogenderivat in einer Menge von 5 bis 100 mg/l zugesetzt. Die Behandlung mit dem Fibrinogenderivat wird vorzugsweise bei 4 bis 40°C durchgeführt. Dabei wird vorzugsweise bei größeren Fibrinogenderivat-Mengen bei niedriger Temperatur, bei kleinen Fibrinogenderivatmengen dagegen bei höherer Temperatur gearbeitet.

Die Inkubationsdauer ist dabei ebenfalls von Temperatur und Menge an zugesetztem Serum oder Plasma und Fibrinogenderivat abhängig. Die Zweikettigkeit von t-PA kann leicht durch an sich bekannte Immunoblotting-Techniken überprüft werden und danach die optimale Inkubationsdauer bestimmt werden.

Im erfindungsgemäßen Verfahren wird in einer bevorzugten Ausführungsform ein mit dem Nachinkubationsmedium identisches Medium zur Kultivierung der eukaryontischen Zellen verwendet.

Das Nachinkubationsmedium, und im Falle der gerade eben genannten Ausführungsform auch das Kulturmedium, enthält vorzugsweise foetales Kälberserum, insbesondere in einer Menge von 0,01 bis 20 %, vorzugsweise in einer Menge von 0,01 bis 10 %.

Die vorzugsweise verwendeten Bedingungen gelten für t-PA-Konzentrationen in der Kultur oder im Kulturüberstand von 5 bis 20 mg/l. Steigt die t-PA-Konzentration bei Beibehaltung der genannten Bedingungen, so steigt gleichzeitig auch der Gehalt an einkettigem t-PA an.

Als Zellen kommen im Rahmen der Erfindung alle in Kultur züchtbaren eukaryontischen Zellen in Betracht, welche t-PA produzieren können. Bevorzugte Beispiele hierfür sind CHO-Zellen (Chinese Hamster Ovary Zellen).

Das folgende Beispiel erläutert die Erfindung weiter:

**Beispiel 1**

a) Transfektion eines das t-PA-Gen enthaltenden Vektors in CHO-Zellen

CHO dhfr⁻-Zellen (ECACC 88072103) werden, wie von R. Kaufmann und P. Sharp, J.Mol.Biol. 15 (1982) 601-621, mit dem Plasmid pSVtPA-dhfr (Gene 66, 193-203 (1988)) transfektiert und stabile Transformanten isoliert.

b) Zellkultur

Die isolierten Kolonien wurden in herkömmlichem Medium (DMEM/F12 im Verhältnis 1:1) mit foetalem Kälberserum (FKS, 0,01 bis 5 %) oder auch serumfrei inkubiert. Die Zeitspanne der Inkubation wurde dabei so gewählt, daß t-PA in quantitativ nachweisbaren Mengen im Medium vorlag, in der Regel erstmals nach 24 Stunden bis theoretisch unbegrenzt. Zur Isolierung von t-PA wurden die t-PA produzierenden Zellen und das t-PA enthaltende Medium voneinander getrennt. Anschließend wurde das t-PA enthaltende Medium mit foetalem Kälberserum (FKS) versetzt und für eine bestimmte Zeitspanne zellfrei inkubiert. Die Inkubation wurde für 20 Stunden durchgeführt, in Tabelle 1 sind die Ausbeuten an ein- und zweikettigem t-PA dargestellt. Hierzu wurde ein Westernblot mit Immunfärbung durchgeführt. Es kann hieraus entnommen werden, daß bereits eine geringe Konzentration an foetalem Kälberserum (0,01 %) ausreicht, um zweikettiges t-PA zu induzieren. Das Verhältnis von ein- und zweikettigem t-PA bleibt unter diesen Bedingungen relativ konstant.

3

Tabelle 1

| % FKS | t-PA | |
|---|---|---|
| | Einkettig | Zweikettig |
| 0 | + | - |
| 0,01 | + | + |
| 0,05 | + | + |
| 0,1 | + | + |
| 0,5 | + | + |
| 1,0 | + | + |
| 2,5 | + | + |
| 5,0 | + | + |

Zu einem 48 Stunden Kulturüberstand von CHO-t-PA-Zellen, die serumfrei kultiviert waren, wurden anschließend 5 % FKS und steigende Konzentrationen von BrCN-Spaltprodukten von Fibrinogen zugegeben und bei 22 °C inkubiert. Das im Kulturüberstand vorhandene t-PA wurde nach Westernblot durch Immunfärbung auf Zweikettigkeit überprüft. Die Resultate sind in Tabelle 2 in Abhängigkeit von der Inkubationszeit dargestellt. Aufgrund dieser Daten ist es ersichtlich, daß durch Fibrinogenderivatzugabe zu serumhaltigen Kulturüberständen die Bildung von praktisch ausschließlich zweikettigem t-PA induziert wird.

Tabelle 2

| Fibrinogenderivat | Zeit (Stunden) | | | | | | | |
|---|---|---|---|---|---|---|---|---|
| | 1 | | 3 | | 5 | | 22 | |
| (mg/ml) | ein | zwei | ein | zwei | ein | zwei | ein | zwei |
| 0 | + | + | + | + | + | + | ( + ) | - |
| 10 | + | + | - | + | - | + | - | + |
| 30 | ( + ) | + | - | + | - | + | - | + |
| 60 | - | + | - | + | - | + | - | + |
| 100 | - | + | - | + | - | + | - | + |

Schließlich wurde untersucht, ob durch Fibrinogen- und FKS-Zugabe und anschließende Inkubation bei 22 °C ein Verlust an t-PA nachweisbar ist. Zu diesem Zweck wurden fibrinogenhaltige Überstände nach Inkubation auf ihre t-PA-Menge durch ELISA untersucht. Die Resultate sind in Tabelle 3 dargestellt.

Tabelle 3

| Fibrinogen | Zeit-Stunden | | | |
|---|---|---|---|---|
| (mg/ml) | 1 | 3 | 5 | 21 |
| | t-PA(mg/ml) | | | |
| 0 | 8 | - | 7 | 7 |
| 10 | 9 | 7 | 8 | 6 |
| 30 | 9 | 8 | 8 | 8 |
| 60 | 10 | 8 | 8 | 7 |
| 100 | 9 | 7 | 10 | 7 |

Aus diesen Ergebnissen wird deutlich, daß die Inkubation von t-PA in Gegenwart von Fibrinogenderivaten und FKS zu keinen gravierenden Unterschieden in der t-PA-Konzentration führte.

**Beispiel 2**

Es wurde analog Beispiel 1 die Hauptkultur durchgeführt, Zellüberstand nach Erreichen der stationären Phase gewonnen und dieser Zellüberstand bei 37° C in Gegenwart von steigenden Mengen Plasmin oder in Gegenwart von 100 mg/l BrCN-Spaltprodukten von Fibrinogen und 5 Vol-% FKS inkubiert.

Tabelle 4 zeigt, daß durch Zusatz von Fibrinogenspaltprodukten und FKS t-PA nicht abgebaut wird und damit eine verlustfreie Umwandlung von einkettigem in zweikettigen t-PA möglich ist. Bei Verwendung von Plasmin zur Umwandlung wird dagegen t-PA abgebaut.

Tabelle 4

| | t-PA Gesamtaktivität (U/ml) | | |
|---|---|---|---|
| | nach Inkubationszeit (h) | | |
| Zusatz von | 0 | 4 | 8 |
| - | 281 | 293 | 285 |
| Plasmin 1,0 U/ml | 281 | 194 | 151 |
| Plasmin 0,1 U/ml | 280 | 267 | 249 |
| Plasmin 0,01 U/ml | 281 | 273 | 235 |
| Plasmin 0,001 U/ml | 279 | 271 | 257 |
| 5 % FKS + 100 mg/l Fibrinogen-Spaltprodukte | 285 | 279 | 281 |

**Beispiel 3**

a) Transfektion von CHO-Zellen mit einem Vektor, der ein t-PA-Muteingen enthält.

Plasmid Pepa144 (hergestellt nach Beispiel 2 von EP A 0 242 836) wird, wie in Beispiel 1a) beschrieben, in CHO-Zellen eingeführt.

Die Kultur der Zellen erfolgt wie in Beispiel 1b) beschrieben. Es wird ebenfalls praktisch ausschließlich zweikettiges t-PA-Mutein erhalten.

**Beispiel 4**

a) Transfektion von CHO dhfr⁻ -Zellen mit einem Vektor, der ein t-PA-Muteingen enthält.

Als t-PA-Muteine werden verwendet $\Delta$K2 und $\Delta$(K2 + EGF), beschrieben in DE 38 25 253 A1.

CHO dhfr⁻-Zellen, ECACC 88072103, wurden wie von Urlaub und Chasin, Proc.Natl.Acad.Soc. USA 77 (1980), 4216-4220 beschrieben, gezüchtet und vermehrt. Zur DNA-Transfektion wurden Calciumphosphat-Präzipitate mit 20 μg pSV ($\Delta$K2)-dhfr (DSM 4721) und pSV$\Delta$(K2 + EGF)-dhfr (DSM 4720), wie von Graham and Van der Eb, Virology 52 (1973), 456-467 beschrieben, in einem Volumen von 4 ml hergestellt. 1 ml des Präzipitats wurde zu $3 \times 10^5$ bis $1 \times 10^6$ Zellen in 10 ml Medium in 9 cm Kulturschalen zugegeben. Die Zellen wurden 8 bis 16 Stunden mit dem Präzipitat inkubiert, dann das Medium entfernt und die Zellen mit 10 ml TBS (25 mmol/l Tris-HCl, pH 7,4, 137 mmol/l NaCl, 5 mmol/l KCl, 0,6 mmol/l $Na_2HPO_4$) gewaschen.

Die Kultivierung der Zellen erfolgt wie in Beispiel 1b) beschrieben. Es werden ebenfalls praktisch ausschließlich zweikettige t-PA-Muteine erhalten.

**Ansprüche**

5

1. Verfahren zur Herstellung von zweikettigem t-PA durch Kultivierung von eukaryontischen Zellen, die mit einem das t-PA-Gen enthaltenden Vektor transfiziert sind, **dadurch gekennzeichnet,** daß man nach Erreichen der stationären Wachstumsphase der Zellen die Kultur oder den Kulturüberstand mit einem Nachinkubationsmedium, welches Serum oder Plasma von Pferd, Rind oder foetalem Kalb und ein Fibrinogenderivat enthält, inkubiert und den t-PA aus dem Medium gewinnt.

2. Verfahren nach Anspruch 1, **dadurch gekennzeichnet,** daß bei der Nachinkubation das Serum oder Plasma in einer Menge von 0,01 bis 20 % vorhanden ist.

3. Verfahren nach Anspruch 1 oder 2, **dadurch gekennzeichnet,** daß man als Fibrinogen-Derivat Spaltprodukte von Fibrinogen verwendet.

4. Verfahren nach Anspruch 3, **dadurch gekennzeichnet,** daß man das Spaltprodukt FCB2 verwendet.

5. Verfahren nach einem der Ansprüche 1 bis 4, **dadurch gekennzeichnet,** daß man das Fibrinogenderivat in einer Menge von 5 bis 100 mg/l einsetzt.

6. Verfahren nach Anspruch 1 bis 5, **dadurch gekennzeichnet,** daß man die Inkubation mit dem Fibrinogenderivat bei 4 bis 40 °C vornimmt.

7. Verfahren nach einem der Ansprüche 1 bis 6, **dadurch gekennzeichnet,** daß zur Kultivierung der Zellen und als Nachinkubation das gleiche Medium verwendet wird.

8. Verfahren nach einem der Ansprüche 1 bis 7, **dadurch gekennzeichnet,** daß das Nachinkubationsmedium und gegebenenfalls auch das Kulturmedium 0,01 bis 10 % foetales Kälberserum enthält.